# EUROPEAN PATENT APPLICATION

(11) **EP 4 764 770 A1**
(43) Date of publication of application: **24.06.2026**
(21) Application number: 24872879.2
(22) Date of filing: 25.09.2024
(51) Int. Cl.: G06F 1/16, G06F 3/01, A61B 5/01, A61B 5/00

(54) **ELECTRONIC DEVICE INCLUDING BATTERY**

(30) Priority: 26.09.2023 KR 20230129276; 30.10.2023 KR 20230147056
(71) Applicant: Samsung Electronics Co., Ltd., Suwon-si, Gyeonggi-do 16677 (KR)
(72) Inventor: JEON, Hwanju, Suwon-si, Gyeonggi-do 16677 (KR)
(74) Representative: HGF
(86) International application number: PCT/KR2024/014440
(87) International publication number: WO 2025/071171

(57) **Abstract**

An electronic device according to an embodiment of the disclosure may include: a housing including a coupling part configured to be be detachably coupled to a ring-shaped external device, wherein the coupling part includes a first coupling surface, a second coupling surface extending from one side of the first coupling surface, a third coupling surface extending from an other side of the first coupling surface, and a connection terminal formed on at least one of the first coupling surface, the second coupling surface, and the third coupling surface and configured to electrically connect the electronic device with the external device based on the electronic device being coupled to the external device, and based on the electronic device being coupled with the external device, the first coupling surface is disposed to face an outer surface facing in a direction opposite to an inner surface of the external device, and the second coupling surface and the third coupling surface are arranged to face a side surface formed between the inner surface and the outer surface of the external device.

## Description

### [Technical Field]

The disclosure relates to an electronic device including a battery.

### [Background Art]

A smart ring-shaped electronic device which can be worn on a part of a user's body may be disposed on an external holding device and receive power from the external holding device. For example, the smart ring-shaped electronic device may be charged using a wireless charging method while being disposed on the external holding device.

The above-described information may be provided as related art for the purpose of aiding understanding of the disclosure. None of the above-described content should be claimed to be prior art related to the disclosure, or should be used to determine prior art.

### [Disclosure of Invention]

A smart ring-shaped electronic device (e.g., a first electronic device, a third electronic device) may be disposed and charged on a holding device, and thus a user may have a difficulty in using the smart ring-shaped electronic device while the smart ring-shaped electronic device is being charged.

A smart ring-shaped electronic device (e.g., the first electronic device, the third electronic device) may be limited in size, and thus battery capacity thereof may be relatively small.

An electronic device according to an example embodiment of the disclosure may include: a housing including a coupling part detachably coupled to a ring-shaped external device, wherein the coupling part includes a first coupling surface, a second coupling surface extending from one side of the first coupling surface, a third coupling surface extending from the other side of the first coupling surface, and a connection terminal formed on at least one of the first coupling surface, the second coupling surface, and the third coupling surface and configured to electrically connect the electronic device with the external device based on the electronic device being coupled to the external device, and based on the electronic device being coupled with the external device, the first coupling surface is disposed to face an outer surface facing in a direction opposite to an inner surface of the external device, and the second coupling surface and the third coupling surface are arranged to face a side surface formed between the inner surface and the outer surface of the external device.

An electronic device according to an example embodiment of the disclosure may include: a ring-shaped housing and a connection terminal formed on at least a portion of a side surface of the housing formed between the inner surface and the outer surface of the housing, and based on the electronic device being disposed on an external device, the side surface of the housing of the electronic device may be disposed to face the side surface of the external device, and the connection terminal may be configured to be electrically connected to the external device.

An electronic device according to an example embodiment of the disclosure may be coupled to a smart ring-shaped electronic device (e.g., the first electronic device, the third electronic device) to transmit power.

A smart ring-shaped electronic device according to an example embodiment of the disclosure may be capable of charging power using an electronic device (e.g., the second electronic device, the fourth electronic device) even while being worn by a user.

An electronic device according to an example embodiment of the disclosure may include a module comprising circuitry capable of performing a specified function, and may provide, in a plug-in manner, a function which is difficult for a smart ring-shaped electronic device (e.g., the first electronic device, the third electronic device) to provide.

### [Brief Description of Drawings]

The above and other aspects, features and advantages of certain embodiments of the present disclosure will be more apparent from the following detailed description, taken in conjunction with the accompanying drawings, in which:
FIG. 1 is a block diagram illustrating an example electronic device in a network environment according to various embodiments;
FIG. 2A and FIG. 2B are diagrams illustrating an example first electronic device and an example second electronic device according to various embodiments;
FIG. 3A and FIG. 3B are perspective views illustrating an example first electronic device and an example second electronic device according to various embodiments;
FIG. 4A and FIG. 4B include a perspective view and a diagram illustrating an example second electronic device according to various embodiments;
FIG. 5 is a perspective view illustrating an example second terminal according to various embodiments;
FIG. 6A and FIG. 6B are perspective views illustrating an example first electronic device and a first terminal according to various embodiments;
FIG. 7A and FIG. 7B are perspective views illustrating an example protrusion according to various embodiments;
FIG. 8 is a diagram illustrating example coupling of a first electronic device and a second electronic device according to various embodiments;
FIG. 9A, FIG. 9B, and FIG. 9C are diagrams illustrating an example coupling part of a second electronic device according to various embodiments;
FIG. 10 is a diagram illustrating example magnetic bodies of a first electronic device and a second electronic device according to various embodiments;
FIG. 11A and FIG. 11B are perspective views illustrating an example third electronic device and an example fourth electronic device according to various embodiments;
FIG. 12 is a diagram illustrating an example fourth electronic device according to various embodiments;
FIG. 13A, FIG. 13B, FIG. 13C, and FIG. 13D include perspective views and diagrams illustrating example magnetic bodies according to various embodiments;
FIG. 14A and FIG. 14B include a perspective view and a diagram illustrating an example third terminal and an example fourth terminal according to various embodiments;
FIG. 15A and FIG. 15B are diagrams illustrating an example third terminal and an example fourth terminal according to various embodiments; and
FIG. 16A, FIG. 16B, FIG. 16C, and FIG. 16D are perspective views illustrating an example third electronic device, fourth electronic device, and holding device according to various embodiments.

### [Mode for the Invention]

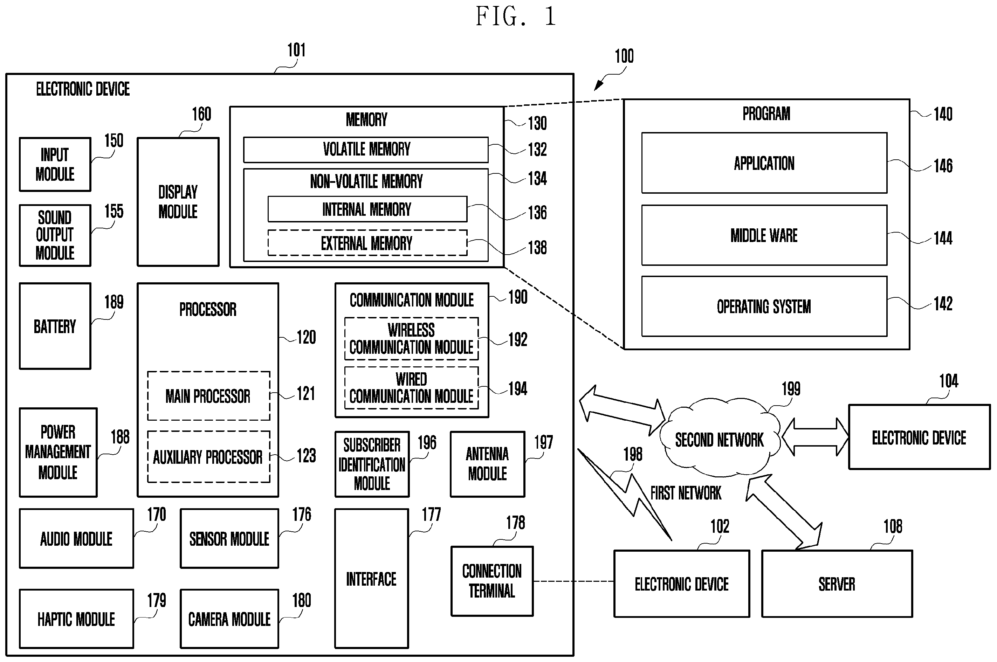

FIG. 1 is a block diagram illustrating an example electronic device 101 in a network environment 100 according to various embodiments. Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In various embodiments, at least one of the components (e.g., the connecting terminal 178) may be omitted from the electronic device 101, or one or more other components may be added in the electronic device 101. In various embodiments, some of the components (e.g., the sensor module 176, the camera module 180, or the antenna module 197) may be implemented as a single component (e.g., the display module 160).

The processor 120 may include various processing circuitry and/or multiple processors. For example, as used herein, including the claims, the term "processor" may include various processing circuitry, including at least one processor, wherein one or more of at least one processor, individually and/or collectively in a distributed manner, may be configured to perform various functions described herein. As used herein, when "a processor", "at least one processor", and "one or more processors" are described as being configured to perform numerous functions, these terms cover situations, for example and without limitation, in which one processor performs some of recited functions and another processor(s) performs other of recited functions, and also situations in which a single processor may perform all recited functions. Additionally, the at least one processor may include a combination of processors performing various of the recited /disclosed functions, e.g., in a distributed manner. At least one processor may execute program instructions to achieve or perform various functions. The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element including a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In an embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

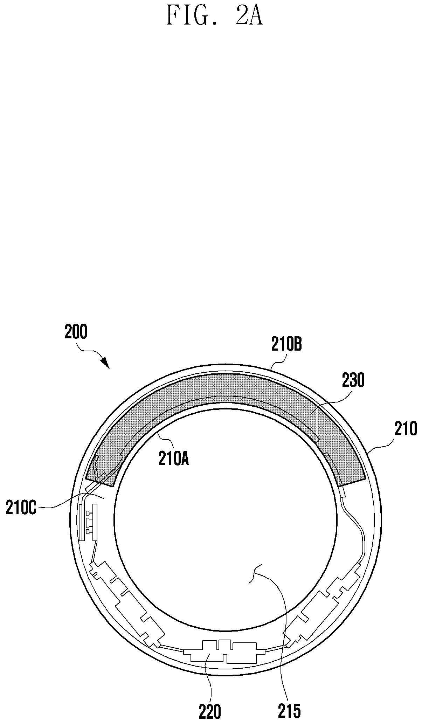

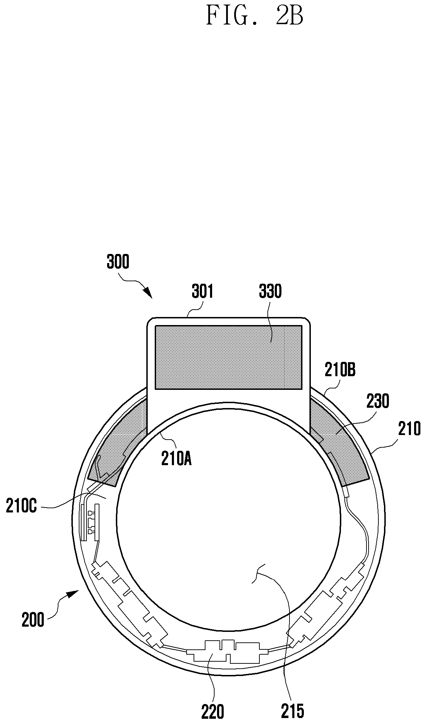

FIG. 2A and FIG. 2B are diagrams illustrating an example first electronic device 200 and second electronic device 300 according to various embodiments.

FIG. 2A is a diagram illustrating the first electronic device 200 according to various embodiments. FIG. 2B is a diagram illustrating the second electronic device 300 disposed on the first electronic device 200 according to various embodiments.

Each of the first electronic device 200 and the second electronic device 300 illustrated in FIG. 2A and FIG. 2B may refer to an electronic device 101 in FIG. 1, or may include at least a part of elements of the electronic device 101 in FIG. 1.

In an embodiment, the first electronic device 200 may be a device which is wearable on at least a part of a user's body. For example, the first electronic device 200 may be a device having a ring shape so that the first electronic device 200 can be worn on a part of the user's body.

Referring to FIG. 2A, the first electronic device 200 may include a first housing 210, a printed circuit board 220, and/or a first battery 230.

In an embodiment, the first housing 210 may configure the exterior of the first electronic device 200. In an embodiment, the first housing 210 may be formed to extend in a ring shape. For example, the first housing 210 may extend along the circumference of a circle having a predetermined radius.

In an embodiment, the first housing 210 may include a first insertion opening 215, an inner surface 210A facing the first insertion opening 215, an outer surface 210B facing in a direction opposite to the inner surface 210A, and/or a side surface 210C formed between the inner surface 210A and the outer surface 210B.

Another element of the first electronic device 200 may be disposed in the first housing 210 according to an embodiment. For example, the printed circuit board 220 and the first battery 230 may be arranged in the first housing 210.

In an embodiment, a processor (e.g., a processor 120 in FIG. 1) and/or a memory (e.g., a memory 130 in FIG. 1) may be mounted on the printed circuit board 220. The processor may include, for example, one or more among a central processing unit, an application processor, a graphics processing unit, an image signal processor, a sensor hub processor, or a communication processor. The processor may include various processing circuitry and/or multiple processors. For example, as used herein, including the claims, the term "processor" may include various processing circuitry, including at least one processor, wherein one or more of at least one processor, individually and/or collectively in a distributed manner, may be configured to perform various functions described herein. As used herein, when "a processor", "at least one processor", and "one or more processors" are described as being configured to perform numerous functions, these terms cover situations, for example and without limitation, in which one processor performs some of recited functions and another processor(s) performs other of recited functions, and also situations in which a single processor may perform all recited functions. Additionally, the at least one processor may include a combination of processors performing various of the recited /disclosed functions, e.g., in a distributed manner. At least one processor may execute program instructions to achieve or perform various functions. According to an embodiment, the memory may include, for example, a volatile memory or a non-volatile memory. In an embodiment, a power management integrated circuit (PMIC) may be mounted on the printed circuit board 220.

In an embodiment, the first battery 230 may serve to supply power to at least one element of the first electronic device 200. The first battery 230 may be formed to be at least partially bent along a direction in which the first housing 210 extends.

In an embodiment, the second electronic device 300 may be a device configured to supply power to the first electronic device 200. For example, the second electronic device 300 may be mounted on the first electronic device 200 to transmit power to the first electronic device 200.

In an embodiment, the second electronic device 300 may include a second housing 301 and/or a second battery 330. In an embodiment, the second battery 330 may be disposed inside the second housing 301.

In an embodiment, the second electronic device 300 may be coupled to at least a part of the first electronic device 200. For example, referring to FIG. 2B, the second electronic device 300 may be coupled to a portion of the outer surface 210B of the first electronic device 200.

In an embodiment, in a case where the second electronic device 300 is coupled to the first electronic device 200, power may be transmitted from the second battery 330 of the second electronic device 300 to the first electronic device 200.

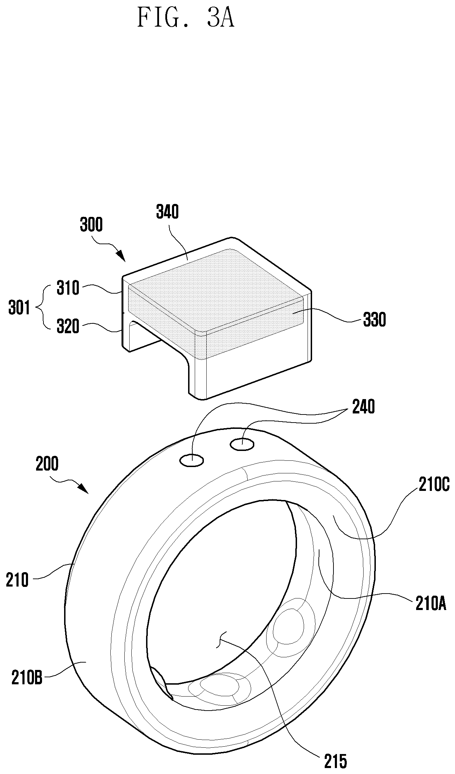

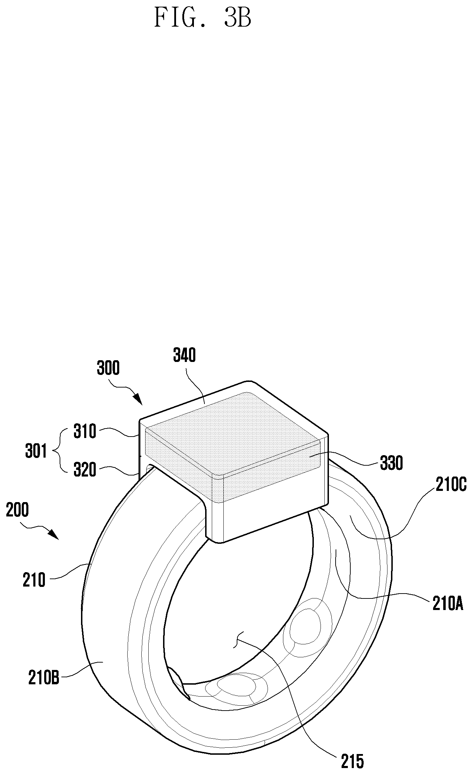

FIG. 3A and FIG. 3B are perspective views illustrating an example first electronic device 200 and second electronic device 300 according to various embodiments.

FIG. 3A is a view illustrating a state in which the first electronic device 200 and the second electronic device 300 are separated from each other according to various embodiments. FIG. 3B is a view illustrating a state in which the second electronic device 300 is disposed on the first electronic device 200 according to various embodiments.

In an embodiment, the first electronic device 200 may include a first terminal 240. The first terminal 240 may be formed in an area of the first electronic device 200 which can be contacted with the second electronic device 300.

In an embodiment, the second electronic device 300 may include a second housing 301, a second battery 330, and/or a display 340.

In an embodiment, the second housing 301 may include a body part 310 and/or a coupling part 320.

In an embodiment, the second battery 330 may be disposed inside the body part 310 of the second housing 301.

In an embodiment, the second electronic device 300 may be coupled with the first electronic device 200 at the coupling part 320 of the second housing 301.

In an embodiment, in a case where the first electronic device 200 and the second electronic device 300 are coupled with each other, the first terminal 240 of the first electronic device 200 may face at least a portion of the coupling part 320 of the second electronic device 300.

In an embodiment, the second electronic device 300 may be disposed in an external charging device (not shown) to receive power. In an embodiment, the second electronic device 300 may be coupled to the first electronic device 200 to transmit power to the first electronic device 200.

In an embodiment, the display 340 may be formed on one surface of the second housing 301. For example, the display 340 may be formed on one surface of the body part 310.

In an embodiment, the display 340 may include a display module 160 in FIG. 1. For example, the display 340 may visually provide information to the outside of the second electronic device 300. The display 340 may include, for example, and without limitation, a hologram device, a projector, etc., and a control circuit configured to control the corresponding device. In an embodiment, the display 340 may include a touch sensor configured to detect a touch, or a pressure sensor configured to measure the intensity of force generated by the touch. In an embodiment, the display 340 may visually display an image to the outside to improve the aesthetics of the second electronic device 300.

In an embodiment, the display 340 may include a material which is visually distinct from other areas of the second electronic device 300. In an embodiment, in a case where the display 340 includes a material which is visually distinct from other areas of the second electronic device 300, the aesthetics of the second electronic device 300 may be improved.

In an embodiment, the second electronic device 300 may include a haptic module (e.g., a haptic module 179 in FIG. 1), a speaker module (e.g., a sound output module 155 in FIG. 1), and/or a microphone module (e.g., an input module 150 in FIG. 1). In an embodiment, the second electronic device 300 may include a module (e.g., a smart key) which can control an external device.

In an embodiment, the second electronic device 300 may include a communication module (e.g., a communication module 190 in FIG. 1) comprising communication circuitry. The second electronic device 300 may be wirelessly connected to an external device (e.g., the first electronic device 200) using the communication module (e.g., the communication module 190 of FIG. 1). In an embodiment, the second electronic device 300 may visually provide, on the display 340, information related to the external device (e.g., the first electronic device 200), the information having been received using the communication module (e.g., the communication module 190 of FIG. 1).

In an embodiment, the second electronic device 300 may include at least one module (e.g., the sound output module 155 in FIG. 1) including various circuitry. Even though the disposition of electronic parts in the first electronic device 200 is limited due to space constraints, the second electronic device 300 may include at least one module capable of performing a predetermined function, so as to provide, in a plug-in manner, a function which is difficult for the first electronic device 200 to provide.

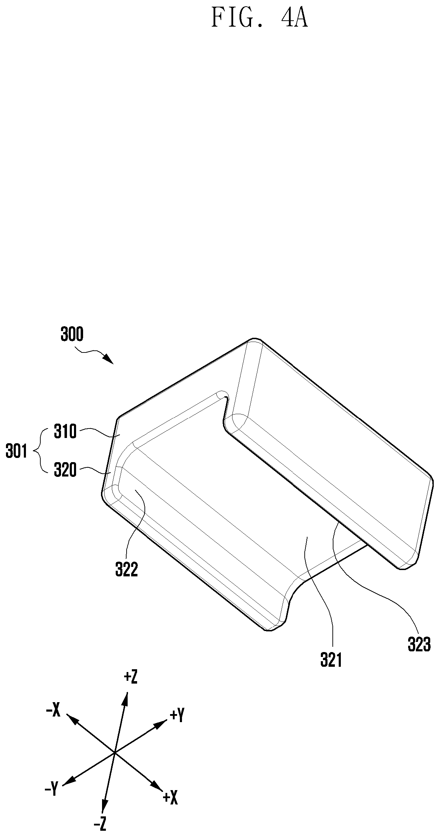

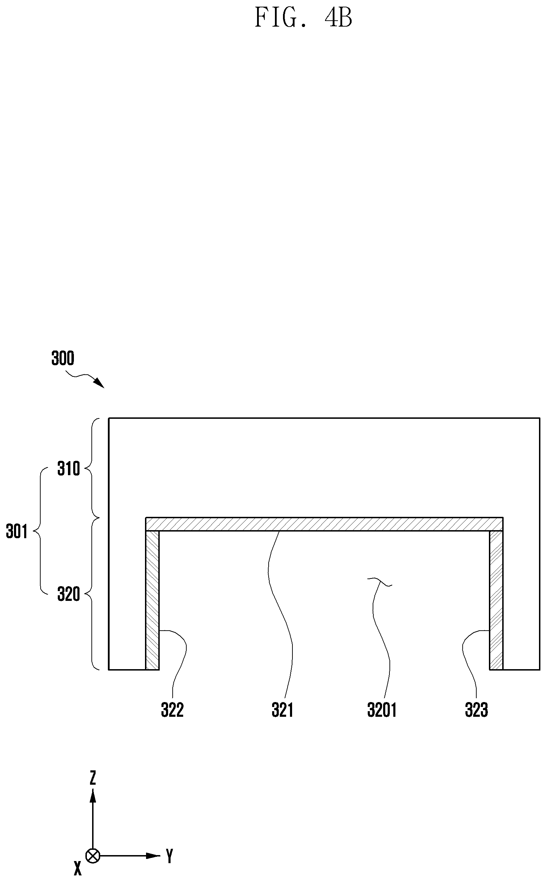

FIG. 4A and FIG. 4B include a perspective view and a diagram illustrating an example second electronic device 300 according to various embodiments.

FIG. 4A is a perspective view of the second electronic device 300 according to various embodiments. FIG. 4B is a diagram illustrating a front view of the second electronic device 300 according to various embodiments.

In describing the second electronic device 300 according to an embodiment of the disclosure, the length direction of the second electronic device 300 may refer to the X-axis direction, and the width direction of the second electronic device 300 may refer to the Y-axis direction. The height direction of the second electronic device 300 may refer to the Z-axis direction.

In an embodiment, the housing 301 of the second electronic device 300 may include a body part 310 and/or a coupling part 320.

In an embodiment, the coupling part 320 may include a first coupling surface 321, a second coupling surface 322, and/or a third coupling surface 323.

Referring to FIG. 4A and FIG. 4B, the first coupling surface 321 according to an embodiment may be a surface facing the height direction (e.g., the negative Z-axis direction) of the second electronic device 300.

In an embodiment, the second coupling surface 322 and the third coupling surface 323 may be formed to be substantially perpendicular to the first coupling surface 321. In an embodiment, each of the second coupling surface 322 and the third coupling surface 323 may be a surface facing the width direction (e.g., the Y-axis direction) of the second electronic device 300.

In an embodiment, the second coupling surface 322 may extend from one side of the first coupling surface 321. For example, referring to FIG. 4A and FIG. 4B, the second coupling surface 322 may extend from one side of the first coupling surface 321 along the height direction of the second electronic device 300.

In an embodiment, the third coupling surface 323 may extend from the other side of the first coupling surface 321, which is opposite to the one side. For example, referring to FIG. 4A and FIG. 4B, the third coupling surface 323 may extend from the other side of the first coupling surface 321 along the height direction of the second electronic device 300.

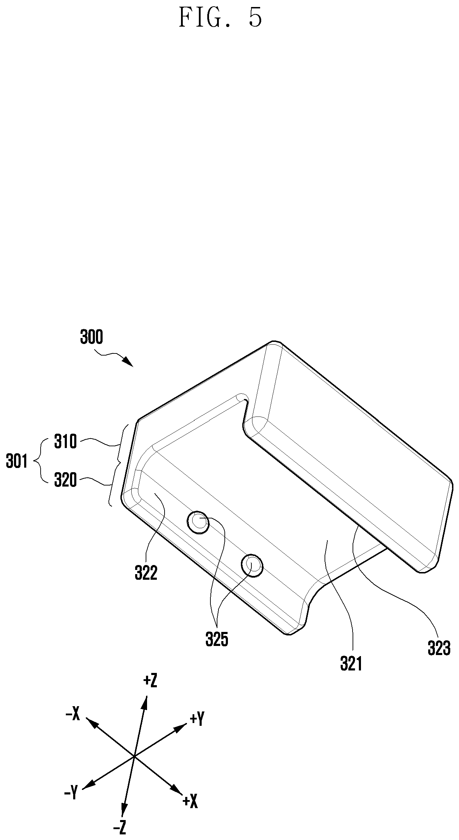

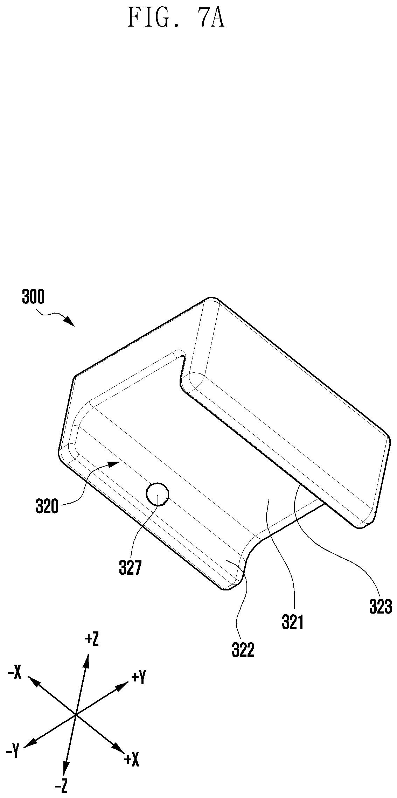

In an embodiment, a second terminal 325 (see FIG. 5) and/or a protrusion 327 (see FIG. 7A) may be formed on at least one of the first coupling surface 321, the second coupling surface 322, and the third coupling surface 323.

In an embodiment, the coupling part 320 may include a coupling space 3201. The coupling space 3201 may be a space surrounded by the first coupling surface 321, the second coupling surface 322, and the third coupling surface 323.

In an embodiment, in a case where the first electronic device 200 (see FIG. 3A) is coupled with the second electronic device 300, at least a part of the first electronic device 200 may be inserted into the coupling space 3201 of the coupling part 320.

In an embodiment, in a case where the first electronic device 200 (see FIG. 3A) is coupled with the second electronic device 300, the outer surface 210B of the first electronic device 200 (see FIG. 3A) may come into contact with the first coupling surface 321 of the coupling part 320.

FIG. 5 is a perspective view illustrating an example second terminal 325 according to various embodiments.

In an embodiment, the coupling part 320 of the second electronic device 300 may include a second terminal 325. The second terminal 325 may be a terminal configured to electrically connect the first electronic device 200 (see FIG. 3A) and the second electronic device 300 to each other.

In an embodiment, the second terminal 325 may be formed to protrude from a portion of the coupling part 320. For example, referring to FIG. 5, the second terminal 325 may be formed to protrude from the second coupling surface 322.

The position of the second terminal 325 shown in FIG. 5 is an example, and the second terminal 325 may be formed on another surface of the coupling part 320. For example, the second terminal 325 may be formed on the first coupling surface 321 and/or the third coupling surface 323 of the coupling part 320.

In an embodiment, the second terminal 325 may be formed on at least one of the first coupling surface 321, the second coupling surface 322, and the third coupling surface 323.

In an embodiment, multiple second terminals 325 may be formed. The multiple second terminals 325 may be formed to be spaced an interval apart from each other. For example, referring to FIG. 5, two second terminals 325 may be formed to be spaced an interval apart from each other.

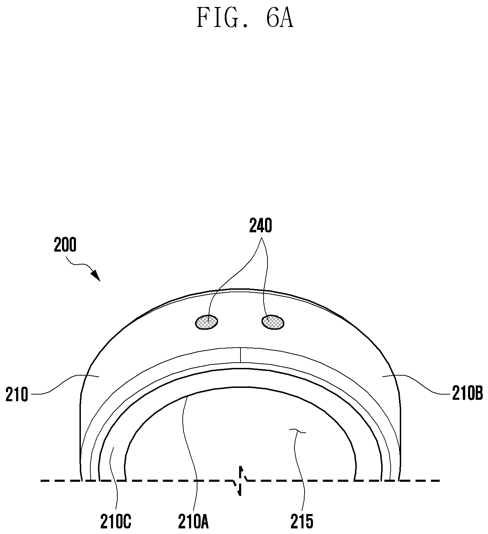

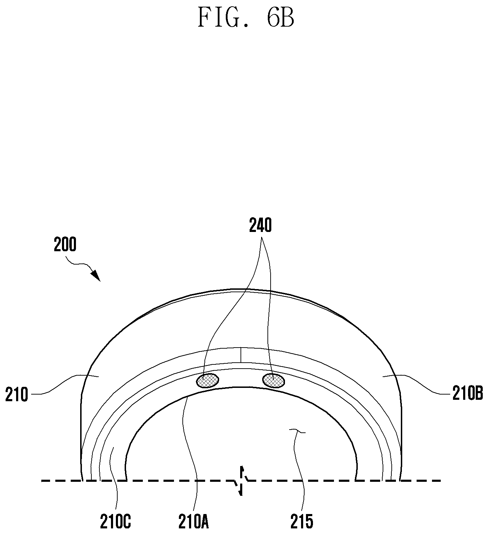

FIG. 6A and FIG. 6B are partial perspective views illustrating an example first electronic device 200 and first terminal 240 according to various embodiments.

FIG. 6A is a perspective view illustrating the first terminal 240 formed on the outer surface 210B of the first electronic device 200 according to various embodiments. FIG. 6B is a perspective view illustrating the first terminal 240 formed on the side surface 210C of the first electronic device 200 according to various embodiments.

In an embodiment, the first electronic device 200 may include a first terminal 240. In an embodiment, the first terminal 240 may be exposed to the outside of the first housing 210.

In an embodiment, the first terminal 240 may be formed on at least one of the outer surface 210B and the side surface 210C of the first housing 210. For example, referring to FIG. 6A, the first terminal 240 according to an embodiment may be exposed to the outside on the outer surface 210B of the first housing 210. Referring to FIG. 6B, the first terminal 240 according to an embodiment may be exposed to the outside on the side surface 210C of the first housing 210. The first terminal 240 according to an embodiment may be formed on each of the outer surface 210B and the side surface 210C of the first housing 210.

In an embodiment, the first terminal 240 may be formed at a position which can be in contact with the second terminal 325 (see FIG. 5) of the second electronic device 300 (see FIG. 5). For example, in a case where the second terminal 325 (see FIG. 5) of the second electronic device 300 (see FIG. 5) is formed at a position where the second terminal can come into contact with the outer surface 210B of the first housing 210 of the first electronic device 200 (e.g., a case where the second terminal 325 (see FIG. 5) is formed on the first coupling surface 321 (see FIG. 5)), the first terminal 240 may be exposed to the outside on the outer surface 210B of the first housing 210. In a case where the second terminal 325 (see FIG. 5) of the second electronic device 300 (see FIG. 5) is formed at a position where the second terminal can come into contact with the side surface 210C of the first housing 210 of the first electronic device 200 (e.g., a case where the second terminal 325 (see FIG. 5) is formed on the second coupling surface 322 (see FIG. 5) and/or the third coupling surface 323 (see FIG. 5)), the first terminal 240 may be exposed to the outside on the side surface 210C of the first housing 210.

In an embodiment, multiple first terminals 240 may be formed. The multiple first terminals 240 may be formed to be spaced an interval apart from each other. For example, referring to FIG. 6A and FIG. 6B, two first terminals 240 may be formed to be spaced an interval apart from each other.

In an embodiment, in a case where the first electronic device 200 is coupled with the second electronic device 300 (see FIG. 5), the first terminal 240 may be in contact with the second terminal 325 (see FIG. 5). Power may be transmitted from the second electronic device 300 (see FIG. 5) to the first electronic device 200, while the first terminal 240 is in contact with the second terminal 325 (see FIG. 5).

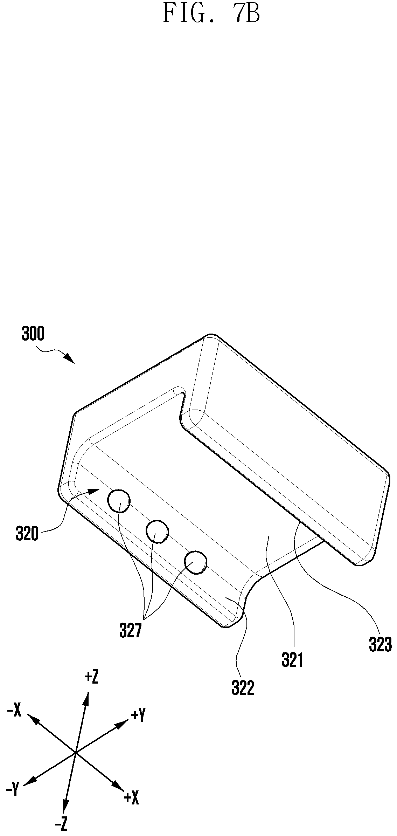

FIG. 7A and FIG. 7B are perspective views illustrating a protrusion 327 according to various embodiments.

FIG. 7A is a perspective view illustrating a second electronic device 300 including one protrusion 327 according to various embodiments. FIG. 7B is a perspective view showing a second electronic device 300 including multiple protrusions 327 according to various embodiments.

In an embodiment, the second electronic device 300 may include a protrusion 327. The second electronic device 300 may be fixed to a predetermined position in the first electronic device 200 (see FIG. 3A) via the protrusion 327.

In an embodiment, in a case where the first electronic device 200 (see FIG. 3A) and the second electronic device 300 are coupled to each other, the protrusion 327 may be inserted into at least a portion of the first electronic device 200 (see FIG. 3A) and the second electronic device 300 may be fixed to a predetermined position in the first electronic device 200 (see FIG. 3A).

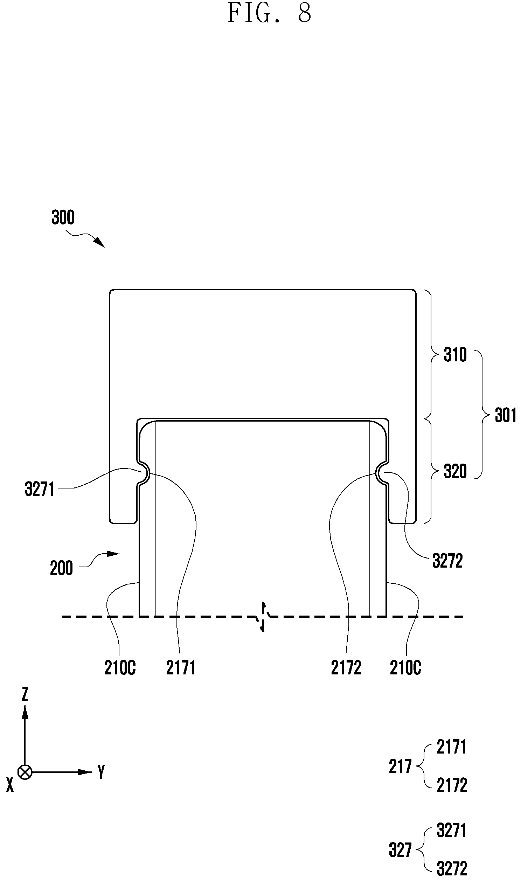

In an embodiment, the first electronic device 200 (see FIG. 3A) may include insertion grooves 217 (see FIG. 8) into which the protrusion 327 of the second electronic device 300 may be inserted.

In an embodiment, the protrusion 327 may be formed to protrude from a portion of the coupling part 320. The protrusion 327 may be formed to protrude from at least one among the surfaces of the coupling part 320. For example, referring to FIG. 7A and FIG. 7B, the protrusion 327 may be formed to protrude from the second coupling surface 322.

The position of the protrusion 327 shown in FIG. 7A and FIG. 7B is an example, and the protrusion 327 may be formed on other surfaces of the coupling part 320. For example, the protrusion 327 may be formed on the first coupling surface 321 and/or the third coupling surface 323 of the coupling part 320.

In an embodiment, multiple protrusions 327 may be formed. For example, referring to FIG. 7B, multiple protrusions 327 may be formed on the coupling part 320. The multiple protrusions 327 may be arranged to be spaced an interval apart from each other.

In an embodiment, the first electronic device 200 (see FIG. 3A) may include multiple insertion grooves 217 (see FIG. 8) into which the multiple protrusions 327 of the second electronic device 300 can be inserted.

In an embodiment, the protrusion 327 may include an elastic material. For example, the protrusion 327 may include an elastic material to be elastically deformed by external force.

In an embodiment, the protrusion 327 may be integrally formed with the second terminal 325 (see FIG. 5). The protrusion 327 according to an embodiment may be integrally formed with the second terminal 325 (see FIG. 5) and may serve to electrically connect the second electronic device 300 and the first electronic device 200 (see FIG. 3A) to each other. In a case where the protrusion 327 and the second terminal 325 (see FIG. 5) are formed integrally, the first terminal 240 (see FIG. 6B) may be formed in an area where the first terminal comes into contact with the protrusion 327 in the first electronic device 200 (see FIG. 6B).

In an embodiment, the second electronic device 300 may include a slide protrusion structure (not shown). For example, the second electronic device 300 may extend in one direction (e.g., the X-axis direction), and may include a slide protrusion structure (not shown) protruding beyond the coupling surfaces 321, 322, and 323. In a case where the second electronic device 300 includes the slide protrusion structure (not shown), the first electronic device 200 (see FIG. 6B) may have a slide groove (not shown) in an area where the slide groove comes into contact with the slide protrusion structure (not shown). When the second electronic device 300 according to an embodiment is coupled to the first electronic device 200 (see FIG. 6B), the slide protrusion structure (not shown) of the second electronic device 300 may be coupled to the slide groove (not shown) of the first electronic device 200 (see FIG. 6B) in a sliding manner.

FIG. 8 is a diagram illustrating example coupling of a first electronic device 200 and a second electronic device 300 according to various embodiments.

Referring to FIG. 8, the first electronic device 200 according to an embodiment may include insertion grooves 217. In an embodiment, the insertion grooves 217 may be formed in the side surface 210C of the housing 210 of the first electronic device 200.

In an embodiment, the insertion grooves 217 may include a first insertion groove 2171 and/or a second insertion groove 2172. The first insertion groove 2171 may be a groove formed on the side surface 210C facing the negative Y-axis direction in the first electronic device 200. The second insertion groove 2172 may be a groove formed on the side surface 210C facing the positive Y-axis direction in the first electronic device 200.

Referring to FIG. 8, the second electronic device 300 according to an embodiment may include protrusions 327. In an embodiment, the protrusions 327 may protrude from the second coupling surface 322 and the third coupling surface 323, respectively.

In an embodiment, the protrusions 327 may include a first protrusion 3271 and/or a second protrusion 3272. The first protrusion 3271 may protrude from the second coupling surface 322 in the positive Y-axis direction. The second protrusion 3272 may protrude from the third coupling surface 323 in the negative Y-axis direction.

In an embodiment, in a case where the first electronic device 200 is coupled with the second electronic device 300, the protrusions 327 of the second electronic device 300 may be inserted into the insertion grooves 217 of the first electronic device 200. For example, the first protrusion 3271 may be inserted into the first insertion groove 2171. The second protrusion 3272 may be inserted into the second insertion groove 2172.

In an embodiment, in a case where the second electronic device 300 is coupled with the first electronic device 200, the protrusions 327 of the second electronic device 300 may be inserted into the insertion grooves 217, and the second electronic device 300 may be fixed to the first electronic device 200.

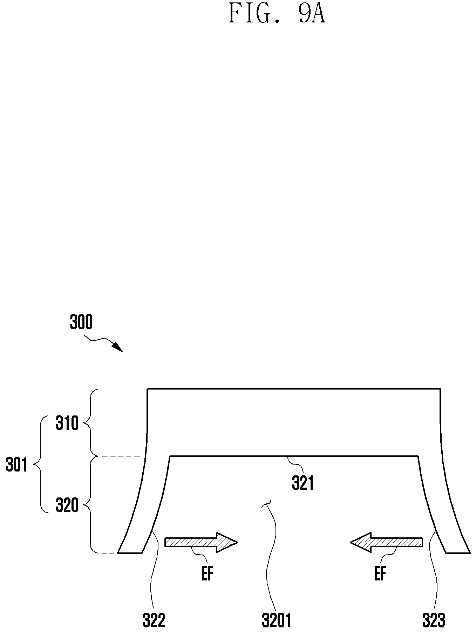

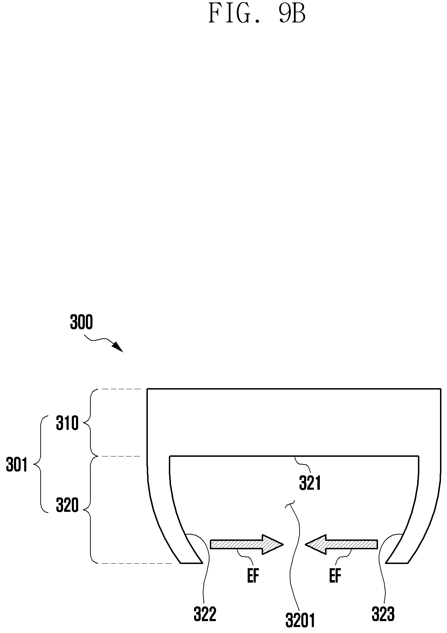

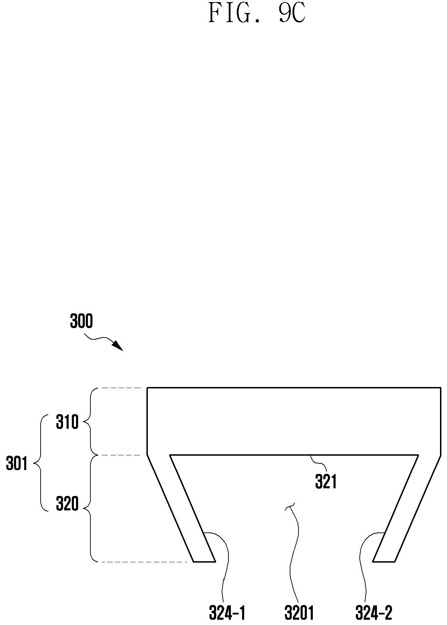

FIG. 9A, FIG. 9B, and FIG. 9C are diagrams illustrating an example coupling part 320 of a second electronic device 300 according to various embodiments.

FIG. 9A and FIG. 9B are diagrams illustrating the second electronic device 300 including an elastically deformable coupling part 320.

FIG. 9C is a diagram illustrating a second coupling surface 324-1 and a third coupling surface 324-2 formed to be inclined according to various embodiments.

In an embodiment, a part of the second electronic device 300 may be elastically deformed. For example, the coupling part 320 of the second electronic device 300 may be elastically deformed.

In an embodiment, the coupling part 320 of the second electronic device 300 may include an elastic material. For example, a portion of the coupling part 320 may be formed of an elastic material to be elastically deformed by external force.

In an embodiment, a second coupling surface 322 and a third coupling surface 323 may include an elastic material to be elastically deformed by external force.

Referring to FIG. 9A, the second coupling surface 322 and the third coupling surface 323 may be elastically deformed in a direction away from each other. In a case where the second coupling surface 322 and the third coupling surface 323 are deformed in a direction away from each other, an elastic force (EF) may be applied to the second coupling surface 322 and the third coupling surface 323 in a direction in which the surfaces approach each other.

Referring to FIG. 9B, the second coupling surface 322 and the third coupling surface 323 may be deformed again in a direction in which the surfaces approach each other by the elastic force (EF) acting in a direction in which the surfaces approach each other.

In an embodiment, a part of the second electronic device 300 may be electrically deformed, and thus the second electronic device 300 may be easily coupled with the first electronic device 200 (see FIG. 3A). For example, while the second coupling surface 322 and the third coupling surface 323 are elastically deformed in a direction away from each other, the coupling space 3201 between the second coupling surface 322 and the third coupling surface 323 is enlarged such that a part of the first electronic device 200 (see FIG. 3A) may be easily inserted into the second electronic device 300.

In an embodiment, a part of the second electronic device 300 may be elastically deformed, and thus the second electronic device 300 may be easily fixed to the first electronic device 200 (see FIG. 3A). For example, while the second coupling surface 322 and the third coupling surface 323 are elastically deformed in a direction in which the surfaces approach each other, the second coupling surface 322 and the third coupling surface 323 may be in close contact with the first electronic device 200 (see FIG. 3A) such that the second electronic device 300 may be fixed to the first electronic device 200 (see FIG. 3A).

Referring to FIG. 9C, the coupling surfaces (e.g., 324-1 and 324-2) of the coupling part 320 according to an embodiment may be formed to be inclined. For example, the second coupling surface 324-1 and the third coupling surface 324-2 according to an embodiment may be formed to be inclined with reference to a plane substantially perpendicular to the first coupling surface 321. In an embodiment, the second coupling surface 324-1 and the third coupling surface 324-2 may be formed to be inclined in a direction (e.g., a direction toward the coupling space 3201) toward the side surface 210C (see FIG. 3A) of the first electronic device 200 (see FIG. 3A).

In an embodiment, the second coupling surface 324-1 and the third coupling surface 324-2 illustrated in FIG. 9C may not include an elastic material. For example, the second coupling surface 324-1 and the third coupling surface 324-2 according to an embodiment may extend in one direction (e.g., an inclined direction with reference to a plane substantially perpendicular to the first coupling surface 321) and may not be elastically deformed.

A part of the first electronic device 200 (see FIG. 3A) according to an embodiment may be inserted into and fixed to the second electronic device 300. For example, a part of the first electronic device 200 (see FIG. 3A) may be inserted into the coupling space 3201 between the second coupling part 324-1 and the third coupling surface 324-2 formed to be inclined and may be fixed to the second electronic device 300.

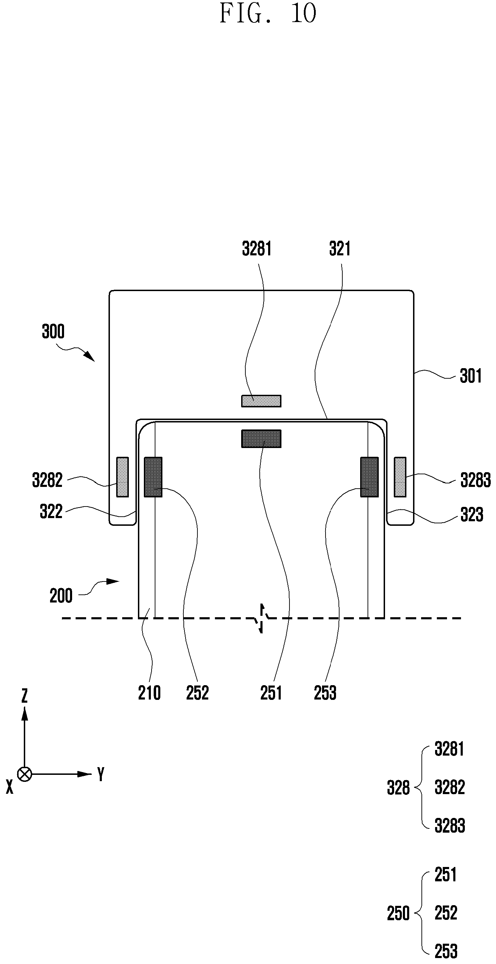

FIG. 10 is a diagram illustrating magnetic bodies 250 and 328 of a first electronic device 200 and a second electronic device 300 according to various embodiments.

In an embodiment, the first electronic device 200 and the second electronic device 300 may include the magnetic bodies 250 and 328. The first electronic device 200 may be coupled with the second electronic device 300 using the magnetic bodies 250 and 328.

In an embodiment, the first electronic device 200 may include at least one first magnetic body 250. The first magnetic bodies 250 of the first electronic device 200 may be arranged inside a first housing 210 of the first electronic device 200.

In an embodiment, the first magnetic bodies 250 may include a (1-1)th magnetic body 251, a (1-2)th magnetic body 252, and/or a (1-3)th magnetic body 253.

In an embodiment, the second electronic device 300 may include at least one second magnetic body 328. The second magnetic bodies 328 of the second electronic device 300 may be arranged inside a second housing 301 of the second electronic device 300.

In an embodiment, the second magnetic bodies 328 may include a (2-1)th magnetic body 3281, a (2-2)th magnetic body 3282, and/or a (2-3)th magnetic body 3283. The (2-1)th magnetic body 3281 may be disposed to face a first coupling surface 321. The (2-2)th magnetic body 3282 may be disposed to face a second coupling surface 322. The (2-3)th magnetic body 3283 may be disposed to face a third coupling surface 323.

In an embodiment, in a case where the second electronic device 300 is coupled with the first electronic device 200, the second magnetic bodies 328 of the second electronic device 300 may be arranged at positions corresponding to the first magnetic bodies 250 of the first electronic device 200. For example, the (2-1)th magnetic body 3281 may be disposed at a position corresponding to the (1-1)th magnetic body 251. The (2-2)th magnetic body 3282 may be disposed at a position corresponding to the (1-2)th magnetic body 252. The (2-3)th magnetic body 3283 may be disposed at a position corresponding to the (1-3)th magnetic body 253.

In an embodiment, in a case where the second electronic device 300 is coupled with the first electronic device 200, the second electronic device 300 may be fixed to the first electronic device 200 through the attractive force produced between the second magnetic bodies 328 of the second electronic device 300 and the first magnetic bodies 250 of the first electronic device 200.

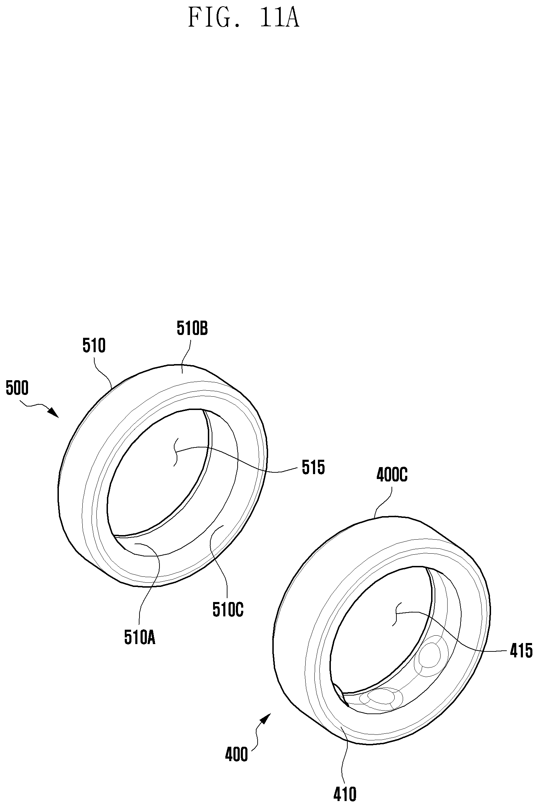

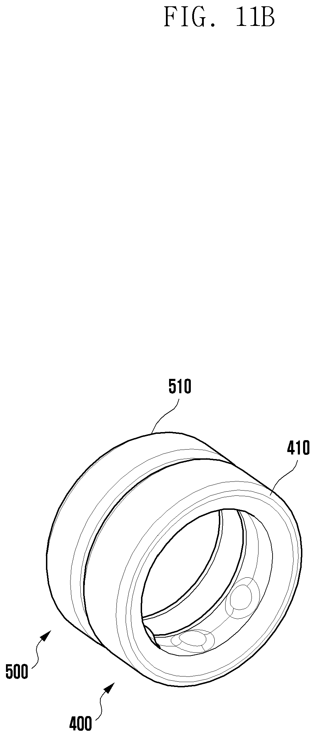

FIG. 11A and FIG. 11B are perspective views illustrating an example third electronic device 400 and fourth electronic device 500 according to various embodiments.

FIG. 11A is a perspective view illustrating a state in which the third electronic device 400 and the fourth electronic device 500 are positioned at a distance according to various embodiments. FIG. 11B is a perspective view illustrating the fourth electronic device 500 disposed on the third electronic device 400 according to various embodiments.

In an embodiment, the third electronic device 400 may be a device which can be worn on at least a part of a user's body. For example, the third electronic device 400 may be a device having a ring shape to be wearable on a part of the user's body.

In an embodiment, the third electronic device 400 may indicate the first electronic device 200, or may include at least a part of the elements of the first electronic device 200.

In an embodiment, the third electronic device 400 may include a third housing 410. Another element (e.g., a third battery (not shown)) of the third electronic device 400 may be disposed in the third housing 410. In an embodiment, the third housing 410 may extend in a circular shape surrounding a second insertion opening 415.

In an embodiment, the fourth electronic device 500 may be a device configured to supply power to the third electronic device 400.

In an embodiment, the fourth electronic device 500 may be formed to have substantially the same shape as the third electronic device 400. For example, the fourth electronic device 500 may have a ring shape.

In an embodiment, the fourth electronic device 500 may include a fourth housing 510. In an embodiment, the fourth housing 510 of the fourth electronic device 500 may include an inner surface 510A facing a third insertion opening 515, an outer surface 510B facing in a direction opposite to the inner surface, and/or a side surface 510C extending between the inner surface 510A and the outer surface 510B.

In an embodiment, a part of the user's body may be inserted into the second insertion opening 415 and/or the third insertion opening 515.

In an embodiment, the fourth housing 510 may include a material which is visually distinct from the third housing 410. In an embodiment, in a case where the fourth housing 510 includes a material which is visually distinct from the third housing 410, the aesthetics of the fourth electronic device 500 may be improved.

Referring to FIG. 11B, the fourth electronic device 500 is disposed in one direction of the third electronic device 400 to transmit power to the third electronic device 400. For example, the fourth electronic device 500 may be disposed so that the side surface 510C of the fourth electronic device 500 faces the side surface 400C of the third electronic device 400 in one direction of the third electronic device 400 to transmit power to the third electronic device 400.

In an embodiment, the fourth electronic device 500 may include a sensor module (e.g., a sensor module 176 in FIG. 1), a haptic module (e.g., the haptic module 179 in FIG. 1), a speaker module (e.g., the sound output module 155 in FIG. 1), and/or a microphone module (e.g., the input module 150 in FIG. 1). In an embodiment, the fourth electronic device 500 may include a module (e.g., a smart key) which can control an external device.

In an embodiment, the fourth electronic device 500 may measure the body temperature of a user wearing the fourth electronic device 500 using the sensor module (e.g., the sensor module 176 in FIG. 1).

In an embodiment, the fourth electronic device 500 may include at least one module (e.g., the sound output module 155 in FIG. 1). Even though the disposition of electronic parts in the third electronic device 400 is limited due to space constraints, the fourth electronic device 500 may include at least one module capable of performing a predetermined function, so as to provide, in a plug-in manner, a function which is difficult for the third electronic device 400 to provide.

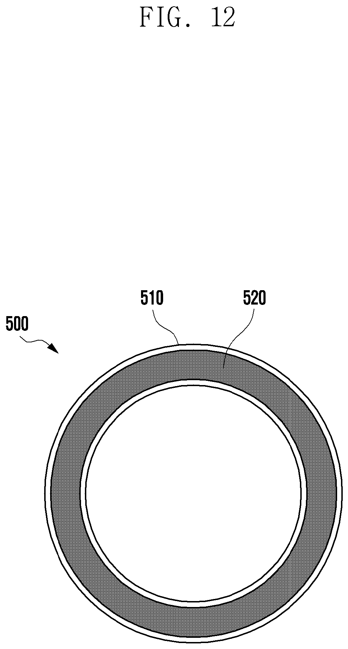

FIG. 12 is a diagram illustrating an example fourth electronic device 500 according to various embodiments.

In an embodiment, the fourth electronic device 500 may include a fourth battery 520 disposed inside a fourth housing 510.

In an embodiment, the fourth battery 520 may be formed such that at least a portion of the fourth battery 520 is bent along a direction in which the fourth housing 510 extends.

In FIG. 12, the fourth battery 520 is shown to extend along the entire area of the fourth housing 510. However, this is just an example, and the fourth battery 520 may be formed to be bent only in a partial area of the fourth housing 510.

In an embodiment, in a case where the fourth electronic device 500 is disposed in one direction of the third electronic device 400, power may be transmitted from the fourth battery 520 of the fourth electronic device 500 to the third electronic device 400.

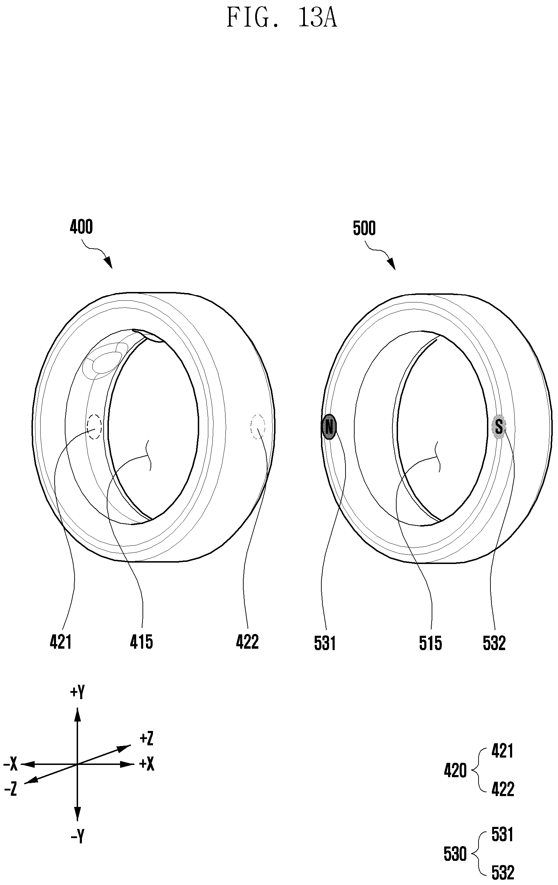

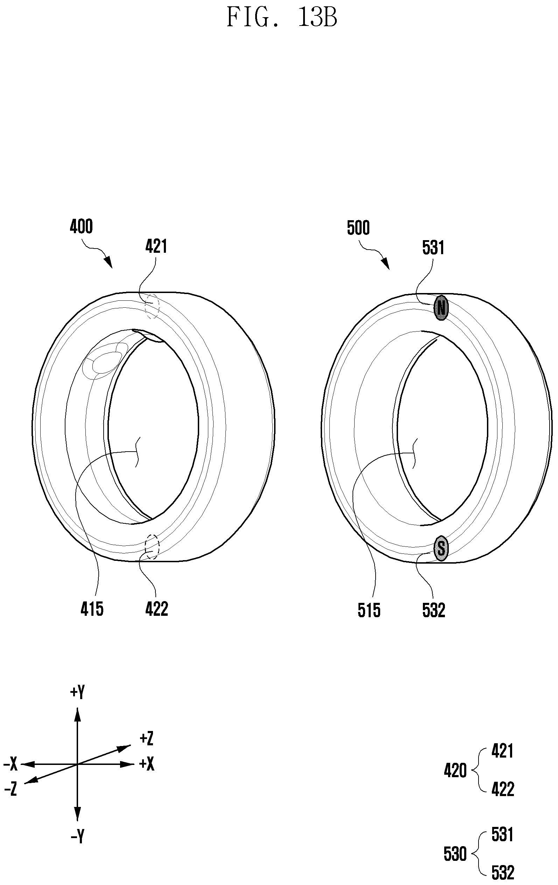

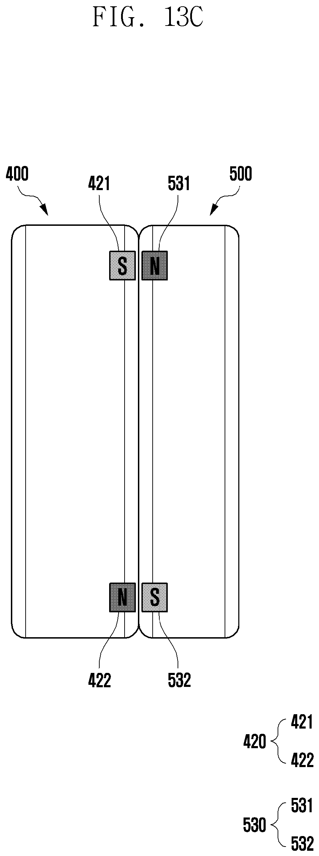

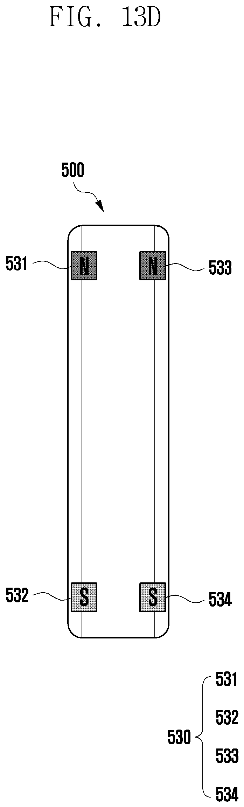

FIG. 13A, FIG. 13B, FIG. 13C, and FIG. 13D includes perspective views an diagrams illustrating example magnetic bodies 420 and 530 according to various embodiments.

FIG. 13A and FIG. 13B are perspective views illustrating the magnetic bodies 420 and 530 according to various embodiments. FIG. 13C is a diagram illustrating a state in which a fourth electronic device 500 is disposed on the third electronic device 400 according to various embodiments.

In an embodiment, the third electronic device 400 may include third magnetic bodies 420. The third magnetic bodies 420 of the third electronic device 400 may include a (3-1)th magnetic body 421 and/or a (3-2)th magnetic body 422.

In an embodiment, the (3-1)th magnetic body 421 and the (3-2)th magnetic body 422 of the third electronic device 400 may be arranged at positions symmetrical to each other around a second insertion opening 415. For example, referring to FIG. 13A, the (3-1)th magnetic body 421 of the third electronic device 400 according to an embodiment may be disposed in a first direction (e.g., the negative X-axis direction) around the second insertion opening 415, and the (3-2)th magnetic body 422 of the third electronic device 400 may be arranged in a second direction (e.g., the positive X-axis direction) around the second insertion opening 415. Referring to FIG. 13B, the (3-1)th magnetic body 421 of the third electronic device 400 according to an embodiment may be disposed in a third direction (e.g., the positive Y-axis direction) around the second insertion opening 415, and the (3-2)th magnetic body 422 of the third electronic device 400 may be disposed in a fourth direction (e.g., the negative Y-axis direction) around the second insertion opening 415.

In an embodiment, the fourth electronic device 500 may include fourth magnetic bodies 530. The fourth magnetic bodies 530 of the fourth electronic device 500 may include a (4-1)th magnetic body 531 and/or a (4-2)th magnetic body 532.

In an embodiment, the (4-1)th magnetic body 531 and the (4-2)th magnetic body 532 of the fourth electronic device 500 may be arranged at positions symmetrical to each other around a third insertion opening 515. For example, referring to FIG. 13A, the (4-1)th magnetic body 531 of the fourth electronic device 500 according to an embodiment may be disposed in a first direction (e.g., the negative X-axis direction) around the third insertion opening 515, and the (4-2)th magnetic body 532 of the fourth electronic device 500 may be disposed in a second direction (e.g., the positive X-axis direction) around the third insertion opening 515. Referring to FIG. 13B, the (4-1)th magnetic body 531 of the fourth electronic device 500 according to an embodiment may be disposed in a third direction (e.g., the positive Y-axis direction) around the third insertion opening 515, and the (4-2)th magnetic body 532 of the fourth electronic device 500 may be disposed in a fourth direction (e.g., the negative Y-axis direction) around the third insertion opening 415.

Referring to FIG. 13A, FIG. 13B, and FIG. 13C, in a case where the fourth electronic device 500 is disposed on the third electronic device 400, the fourth magnetic bodies 530 of the fourth electronic device 500 may be arranged at positions corresponding to the third magnetic bodies 420 of the third electronic device 400. For example, the (4-1)th magnetic body 531 of the fourth electronic device 500 may be disposed at a position corresponding to the (3-1)th magnetic body 421 of the third electronic device 400, and the (4-2)th magnetic body 532 of the fourth electronic device 500 may be disposed at a position corresponding to the (3-2)th magnetic body 422 of the third electronic device 400.

Referring to FIG. 13C, in a case where the fourth electronic device 500 is disposed on the third electronic device 400, different poles of the magnetic bodies 530 and 420 may be arranged to face each other. For example, the N pole of the (4-1)th magnetic body 531 of the fourth electronic device 500 and the S pole of the (3-1)th magnetic body 421 of the third electronic device 400 may be arranged to face each other. The S pole of the (4-2)th magnetic body 532 of the fourth electronic device 500 and the N pole of the (3-2)th magnetic body 422 of the third electronic device 400 may be arranged to face each other. Different poles of the magnetic bodies 530 and 420 may be arranged to face each other, and an attractive force may be produced between the fourth magnetic bodies 530 of the fourth electronic device 500 and the third magnetic bodies 420 of the third electronic device 400.

In an embodiment, in a case where the fourth electronic device 500 is disposed on the third electronic device 400, the fourth electronic device 500 may be fixed to the third electronic device 400 through an attractive force produced between the fourth magnetic bodies 530 of the fourth electronic device 500 and the third magnetic bodies 420 of the third electronic device 400.

Referring to FIG. 13D, a fourth electronic device according to an embodiment may include the fourth magnetic bodies 530 on both sides, respectively. The fourth magnetic bodies 530 according to an embodiment may include a (4-1)th magnetic body 531, a (4-2)th magnetic body 532, a (4-3)th magnetic body 533, and/or a (4- 4)th magnetic body 534. The (4-3)th magnetic body 533 may be disposed to face a surface located in the opposite direction to the surface the (4-1)th magnetic body 531 faces. The (4-4)th magnetic body 534 may be disposed to face a surface located in the opposite direction to the surface the (4-2)th magnetic body 532 faces.

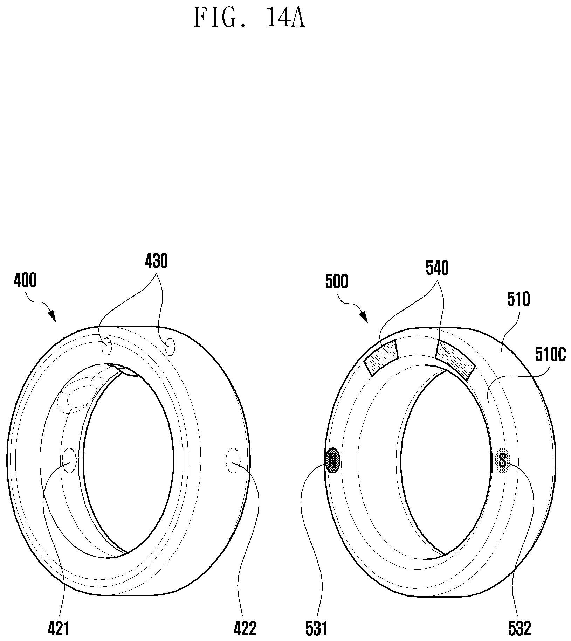

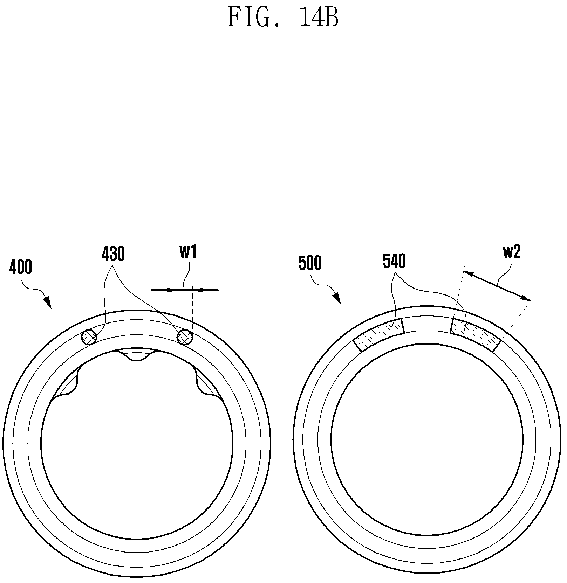

FIG. 14A and FIG. 14B include a perspective view and diagram illustrating a third terminal 430 and a fourth terminal 530 according to various embodiments.

In an embodiment, a third electronic device 400 may include a third terminal 430. The third electronic device 400 may receive power from the fourth electronic device 500 through the third terminal 430.

In an embodiment, the fourth electronic device 500 may include a fourth terminal 530. The fourth electronic device 500 may supply power to the third electronic device 400 through the fourth terminal 540.

In an embodiment, the third terminal 430 may be formed at a position where the third terminal does not overlap with third magnetic bodies 421 and 422 of the third electronic device 400. In an embodiment, the fourth terminal 530 may be formed at a position where the fourth terminal does not overlap fourth magnetic bodies 531 and 532 of the fourth electronic device 500. For example, partial areas of the side surface 510C of the fourth housing 510, which the fourth magnetic bodies 531 and 532 face, may be different from a partial area of the side surface 510C of the fourth housing 510 where the fourth terminal 540 is formed.

In an embodiment, the area occupied by the fourth terminal 540 may be larger than the area occupied by the third terminal 430. For example, referring to FIG. 14B, the third terminal 430 may have a first width W1. The fourth terminal 540 may have a second width W2. The second width W2 may be formed to be longer than the first width W1.

In an embodiment, the area occupied by the fourth terminal 540 is larger than the area occupied by the third terminal 430, and thus when the fourth electronic device 500 is disposed on the third electronic device 400, even though the third terminal 430 and the fourth terminal 540 are not perfectly aligned, it may be easy for the third terminal 430 and the fourth terminal 540 to maintain contact with each other.

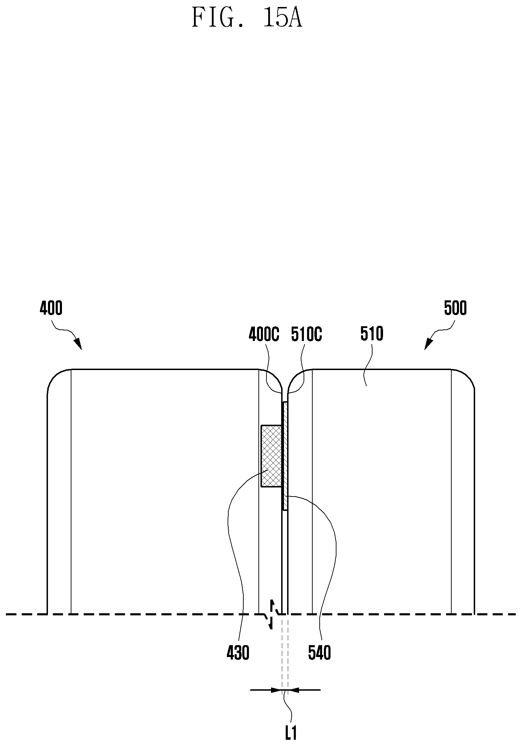

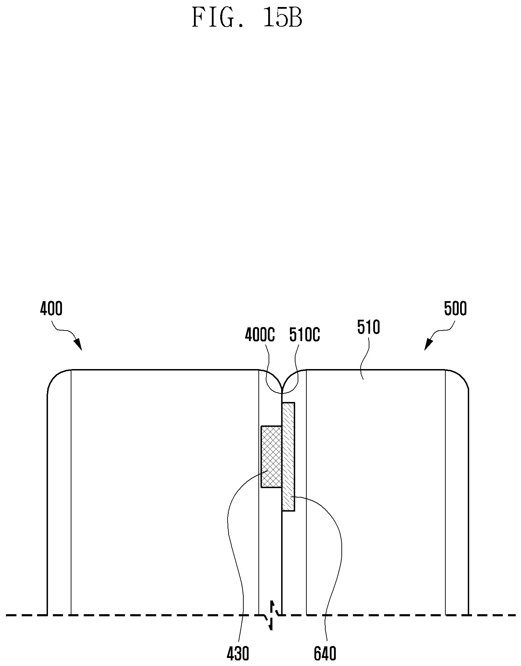

FIG. 15A and FIG. 15B are diagrams illustrating a third terminal 430 and a fourth terminal 540 or 640 according to various embodiments.

FIG. 15A is a diagram illustrating the fourth terminal 540 according to an embodiment. FIG. 15B is a diagram illustrating the fourth terminal 640 according to an embodiment.

In an embodiment, in a case where the fourth electronic device 500 is disposed on the third electronic device 400, the fourth terminal 540 of the fourth electronic device 500 may be in contact with the third terminal 430 of the third electronic device 400.

In an embodiment, while the fourth terminal 540 is in contact with the third terminal 430, power may be transmitted from the fourth electronic device 500 to the third electronic device 400.

Referring to FIG. 15A and FIG. 15B, the third terminal 430 according to an embodiment may configure substantially the same surface as a side surface 400C of the third electronic device 400. By configuring substantially the same surface as the side surface 400C of the third electronic device 400, the third terminal 430 may improve the aesthetics of the third electronic device 400.

Referring to FIG. 15A, the fourth terminal 540 according to an embodiment may be formed to protrude from the side surface 510C of a fourth housing 510 of the fourth electronic device 500. For example, the fourth terminal 530 may protrude from the side surface 510C by a first length L1. In an embodiment, the first length L1 may be approximately 0.1 mm to 0.5 mm.

In an embodiment, in a case where the fourth terminal 540 protrudes from the side surface 510C of the fourth housing 510, when the fourth electronic device 500 is disposed on the third electronic device 400, even if external foreign matters are introduced between the third electronic device 400 and the fourth electronic device 500, the fourth terminal 540 and the third terminal 430 may be easily contacted.

Referring to FIG. 15B, the fourth terminal 640 according to an embodiment may configure substantially the same surface as the side surface 510C of the fourth housing 510 of the fourth electronic device 500.

In an embodiment, in a case where the fourth terminal 640 configures substantially the same surface as the side surface 510C of the fourth electronic device 500, the side surface 510C of the fourth housing 510 may extend on the same plane as a whole, and thus the aesthetics of the fourth electronic device 500 can be improved.

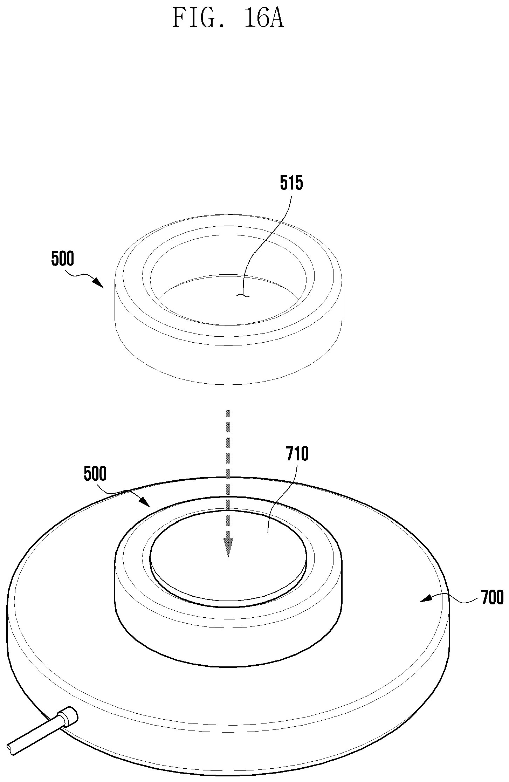

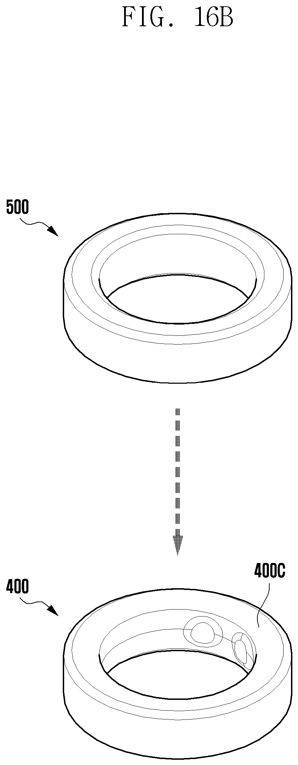

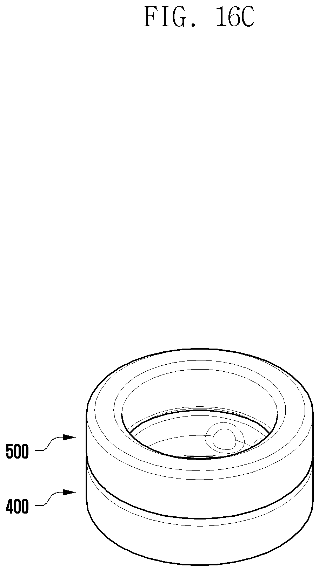

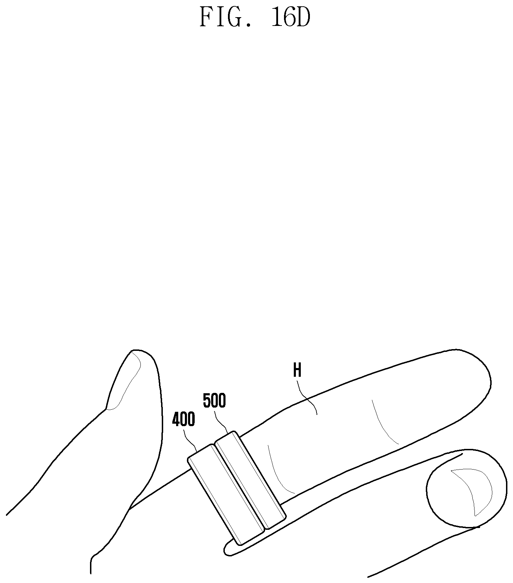

FIG. 16A, FIG. 16B, FIG. 16C, and FIG. 16D are perspective views illustrating a third electronic device 400, a fourth electronic device 500, and an example holding device 700 according to various embodiments.

FIG. 16A is a perspective view illustrating the holding device 700 and the fourth electronic device 500 according to various embodiments. FIG. 16B and FIG. 16C are perspective views illustrating the third electronic device 400 and the fourth electronic device 500 according to various embodiments. FIG. 16D is a perspective view illustrating the third electronic device 400 and the fourth electronic device 500 worn on a part of the user's body.

Referring to FIG. 16A, the fourth electronic device 500 according to an embodiment may be disposed on the holding device 700. In a case where the fourth electronic device 500 is disposed on the holding device 700, a seating part 710 of the holding device 700 may be inserted into an insertion opening 515 of the fourth electronic device 500. The fourth electronic device 500 may be disposed on the holding device 700 to receive power from the holding device 700.

In an embodiment, the fourth electronic device 500 may be disposed on the third electronic device 400. For example, referring to FIG. 16B and FIG. 16C, the fourth electronic device 500 may be located at a distance from the third electronic device 400, and then, may move toward the third electronic device 400 to come into contact with the third electronic device 400. Referring to FIG. 16B and 16C, the fourth electronic device 500 may be disposed in contact with a side surface 400C of the third electronic device 400. While the fourth electronic device 500 is disposed in contact with the third electronic device 400, the fourth electronic device 500 may transmit power to the third electronic device 400.

In an embodiment, the third electronic device 400 and the fourth electronic device 500 may be worn on a part of the user's body. For example, referring to FIG. 16D, while the third electronic device 400 is worn on the user's finger (H), the fourth electronic device 500 may be additionally worn on the user's finger (H). The third electronic device 400 and the fourth electronic device 500 worn on the user's finger (H) may be arranged to be in contact with each other. The fourth electronic device 500 worn on the user's finger (H) can transmit power to the third electronic device 400.

An electronic device (e.g., the second electronic device 300) according to an example embodiment of the disclosure may include a housing 301 including a coupling part configured to be detachably coupled to a ring-shaped external device (e.g. the first electronic device 200).

In an example embodiment, the coupling part 320 may include a first coupling surface 321, a second coupling surface 322 extending from one side of the first coupling surface 321, a third coupling surface 323 extending from an other side of the first coupling surface 321, and a connection terminal (e.g., the second terminal 325) formed on at least one of the first coupling surface 321, the second coupling surface 322, and the third coupling surface 323 and configured to electrically connect the electronic device with the external device 200 based on the electronic device being coupled to the external device 200.

In an example embodiment, based on an electronic device 300 being coupled to the external device 200, the first coupling surface 321 may be disposed to face an outer surface 210B facing in a direction opposite to an inner surface 210A of the external device 200, and the second coupling surface 322 and the third coupling surface 323 may be arranged to face a side surface 210C formed between the inner surface 210A and the outer surface 210B of the external device 200.

In an example embodiment, the second coupling surface 322 and the third coupling surface 323 each include an elastic material, and based on the electronic device 300 being coupled to the external device 200, the external device 200 may be fitted to the electronic device 300 through the elasticity of the second coupling surface 322 and the third coupling surface 323.

In an example embodiment, the coupling part 320 may further include a first protrusion 3271 protruding from the second coupling surface 322 and a second protrusion 3272 protruding from the third coupling surface 323, and based on the electronic device 300 being coupled with the external device, the first protrusion 3271 and the second protrusion 3272 may be inserted into grooves (e.g., the insertion grooves 217 in FIG. 8) of the external device 200 corresponding to the first protrusion 3271 and the second protrusion 3272, respectively.

In an example embodiment, a connection terminal 325 may be integrally formed with the first protrusion 3271 and the second protrusion 3272.

In an example embodiment, the coupling part 320 may include multiple first protrusions 3271 and multiple second protrusions 3272, respectively, the multiple first protrusion 3271 may be formed at intervals with each other on the second coupling surface 322, and the multiple second protrusions 3272 may be formed at intervals with each other on the third coupling surface 323.

In an example embodiment, the coupling part 320 may include one or more magnetic bodies 328 comprising a magnet arranged inside the housing 301, and based on the electronic device 300 being coupled with the external device 200, the electronic device 300 may be fixed to the external device 200 through the attractive force produced by the magnetic bodies 328.

In an example embodiment, the magnetic bodies 328 may include a first magnetic body 3281 facing the first coupling surface 321, a second magnetic body 3282 facing the second coupling surface 322, and a third magnetic body 3283 facing the third coupling surface 323.

In an example embodiment, the electronic device 300 may include a battery 330 disposed inside the housing 301.

In an example embodiment, the housing 301 may include a body part 310 surrounding the battery 330, and the coupling part 320 may be formed in one direction of the body part 310.

In an example embodiment, the external device 200 may have a ring shape, and may be a device which can be worn on at least a part of the user's body.

In an example embodiment, a second coupling surface 324-1 and a third coupling surface 324-2 may be formed to be inclined with reference to a plane perpendicular to the first coupling surface 321 in a direction towards the side surface 210C of the external device 200.

In an example embodiment, the electronic device 300 may include a display 340 formed on one surface of the housing 301, and a speaker module (e.g., the sound output module 155 in FIG. 1) including a speaker disposed inside the housing 301.

An electronic device (e.g., the fourth electronic device 500) according to an example embodiment of the disclosure may include a ring-shaped housing 510 and a connection terminal (e.g., the fourth terminal 530) formed on at least a portion of a side surface 510C formed between an inner surface 510A and an outer surface 510Bof the housing 510.

In an example embodiment, based on an electronic device being disposed on an external device (e.g., the third electronic device 400), a side surface 510C of the housing 510 in the electronic device 500 may be disposed to face a side surface 400C of the external device 400, and the connection terminal 540 may be electrically connected to the external device 400.

In an example embodiment, the electronic device 500 may include one or more magnetic bodies 530 comprising a magnet arranged inside the housing510, and based on the electronic device 500 being disposed on the external device 400, the electronic device 500 can be fixed to the external device 400 through the attractive force produced by the magnetic bodies 530.

In an example embodiment, the magnetic bodies 530 may include a first magnetic body 531 and a second magnetic body 532 disposed at a position symmetrical to the first magnetic body 531 around an insertion opening 515 of the housing 510.

In an example embodiment, the magnetic bodies 530 may be arranged to face a partial area of the side surface 510C of the housing 510, and the partial area of the side surface 510C, which the magnetic bodies 530 face, may be different from a partial area of the side surface 510C of the housing 510 where the connection terminal 540 is formed.

In an example embodiment, the connection terminal 540 may be formed to protrude from the side surface 510C of the housing 510.

In an example embodiment, the connection terminal 540 may configure the same surface as the side surface 510C of the housing 510.

In an example embodiment, based on the electronic device 500 being disposed on the external device 400, the connection terminal 540 may contact a charging terminal (e.g., the third terminal 430) of the external device 400, and the area of the connection terminal 540 may be larger than the area of the charging terminal 430.

In an example embodiment, the electronic device 500 may be a device which can be worn on at least a part of the user's body, and based on the user wearing the electronic device 500, a part of the user's body may be inserted through the insertion opening 515.

In an example embodiment, the external device 400 may have a ring shape, and may be a device which can be worn on at least a part of the user's body.

In an example embodiment, the electronic device 500 may include a battery 520 disposed inside the housing 510.

In an example embodiment, based on the electronic device 500 being disposed on the external device 400, the electronic device 500 may be configured to transmit power produced from the battery 520 to the external device.

In an example embodiment, the electronic device 500 may include a sensor module (e.g., the sensor module 176 in FIG. 1) including a sensor configured to measure the body temperature of a user wearing the electronic device 500.

The electronic device according to an embodiment of the disclosure may be one of various types of electronic devices. The electronic device may include, for example, a portable communication device (e.g., a smart phone), a computer device, a portable multimedia device, a portable medical device, a camera, a wearable device, a home appliance, or the like. The electronic device according to embodiments of the disclosure is not limited to those described above.

It should be appreciated that an embodiment of the disclosure and the terms used therein are not intended to limit the technological features set forth herein to particular embodiments and the disclosure includes various changes, equivalents, or alternatives for a corresponding embodiment. With regard to the description of the drawings, similar reference numerals may be used to designate similar or relevant elements. an item may include one or more of the items, unless the relevant context clearly indicates otherwise. As used herein, each of such phrases as "A or B," "at least one of A and B," "at least one of A or B," "A, B, or C," "at least one of A, B, and C," and "at least one of A, B, or C," may include all possible combinations of the items enumerated together in a corresponding one of the phrases. Such terms as "a first," "a second," "the first," and "the second" may be used to simply distinguish a corresponding element from another, and does not limit the elements in other aspect (e.g., importance or order). If an element (e.g., a first element) is referred to, with or without the term "operatively" or "communicatively", as "coupled with/to" or "connected with/to" another element (e.g., a second element), the element may be coupled/connected with/to the other element directly (e.g., wiredly), wirelessly, or via a third element.

As used in an embodiment of the disclosure, the term "module" may include a unit implemented in hardware, software, or firmware, or any combination thereof, and may be interchangeably used with other terms, for example, "logic," "logic block," "component," or "circuit". The "module" may be a single integrated component, or a minimum unit or part thereof, adapted to perform one or more functions. For example, according to an embodiment, the "module" may be implemented in the form of an application-specific integrated circuit (ASIC).

An embodiment of the disclosure may be implemented as software (e.g., the program 101) including one or more instructions that are stored in a storage medium (e.g., the internal memory 136 or external memory 138) that is readable by a machine (e.g., the electronic device 140). For example, a processor (e.g., the processor 120) of the machine (e.g., the electronic device 101) may invoke at least one of the one or more instructions stored in the storage medium, and execute it. This allows the machine to be operated to perform at least one function according to the at least one instruction invoked. The one or more instructions each may include a code generated by a compiler or a code executable by an interpreter. The machine-readable storage medium may be provided in the form of a non-transitory storage medium. Wherein, the "non-transitory" storage medium is a tangible device, and does not include a signal (e.g., an electromagnetic wave), but this term does not differentiate between where data is semi-permanently stored in the storage medium and where the data is temporarily stored in the storage medium.

According to an embodiment, methods according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., Play Store ^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each element (e.g., a module or a program) of the above-described elements may include a single entity or multiple entities, and some of the multiple entities may also be separately disposed in another element. According to various embodiments, one or more of the above-described elements or operations may be omitted, or one or more other elements or operations may be added. Alternatively or additionally, a plurality of elements (e.g., modules or programs) may be integrated into a single element. In such a case, according to various embodiments, the integrated element may still perform one or more functions of each of the plurality of elements in the same or similar manner as they are performed by a corresponding one of the plurality of elements before the integration.

According to various embodiments, operations performed by the module, the program, or another element may be carried out sequentially, in parallel, repeatedly, or heuristically, or one or more of the operations may be executed in a different order or omitted, or one or more other operations may be added.

While the disclosure has been illustrated and described with reference to various example embodiments, it will be understood that the various example embodiments are intended to be illustrative, not limiting. It will be further understood by those skilled in the art that various changes in form and detail may be made without departing from the true spirit and full scope of the disclosure, including the appended claims and their equivalents. It will also be understood that any of the embodiment(s) described herein may be used in conjunction with any other embodiment(s) described herein.

## Claims

1. An electronic device (300) comprising a housing (301) comprising a coupling part (320) detachably coupled to a ring-shaped external device (200),
wherein the coupling part comprises:
a first coupling surface (321);
a second coupling surface (322) extending from one side of the first coupling surface;
a third coupling surface (323) extending from an other side of the first coupling surface; and
a connection terminal (325) formed on at least one of the first coupling surface, the second coupling surface, and the third coupling surface and configured to electrically connect the electronic device with the external device based on the electronic device being coupled to the external device,
wherein based on the electronic device being coupled with the external device, the first coupling surface is disposed to face an outer surface (210B) facing in a direction opposite to an inner surface (210A) of the external device, and
wherein the second coupling surface and the third coupling surface are arranged to face side surfaces (210C) formed between the inner surface and the outer surface of the external device.

2. The electronic device of claim 1, wherein each of the second coupling surface and the third coupling surface comprises an elastic material, and
wherein based on the electronic device being coupled with the external device, the external device is configured to be fitted to the electronic device through the elasticity of the second coupling surface and the third coupling surface

3. The electronic device of claim 1, wherein the coupling part further comprises:
a first protrusion (3271) protruding from the second coupling surface; and
a second protrusion (3272) protruding from the third coupling surface, and
wherein based on the electronic device being coupled with the external device, the first protrusion and the second protrusion are configured to be inserted into grooves (217) of the external device corresponding to the first protrusion and the second protrusion.

4. The electronic device of claim 3, wherein the connection terminal is integrally formed with the first protrusion and the second protrusion.

5. The electronic device of claim 3, wherein the coupling part comprises multiple first protrusions and multiple second protrusions, respectively, and
wherein the multiple first protrusions are formed at intervals with each other on the second coupling surface, and the multiple second protrusions are formed at intervals with each other on the third coupling surface.

6. The electronic device of claim 1, wherein the coupling part comprises at least one magnetic body (328)comprising a magnet arranged inside the housing, and
wherein based on the electronic device being coupled with the external device, the electronic device is configured to be fixed to the external device through the attractive force produced by the magnetic body.

7. The electronic device of claim 6, wherein the at least one magnetic body comprises:
a first magnetic body facing the first coupling surface;
a second magnetic body facing the second coupling surface; and
a third magnetic body facing the third coupling surface.

8. The electronic device of claim 1, further comprising a battery disposed inside the housing,
wherein the housing comprises a body part surrounding the battery, and
wherein the coupling part is formed in one direction of the body part.

9. The electronic device of claim 1, wherein the second coupling surface and the third coupling surface are inclined with reference to a plane perpendicular to the first coupling surface in a direction towards the side surfaces of the external device.

10. The electronic device of claim 1, further comprising:
a display disposed on one surface of the housing; and
a speaker module comprising a speaker disposed inside the housing.

11. An electronic device comprising:
a ring-shaped housing; and
a connection terminal formed on at least a portion of a side surface formed between an inner surface and an outer surface of the housing,
wherein based on the electronic device being disposed on an external device, the side surface of the housing is disposed to face a side surface of the external device, and
wherein the connection terminal is configured to be electrically connected to the external device.

12. The electronic device of claim 11, further comprising at least one magnetic body comprising a magnet arranged inside the housing,
wherein based on the electronic device being disposed on the external device, the electronic device is configured to be fixed to the external device through the attractive force produced by the magnetic body.

13. The electronic device of claim 12, wherein the at least one magnetic body comprises:
a first magnetic body comprising a magnet; and
a second magnetic body comprising a magnet disposed at a position symmetrical to the first magnetic body around an insertion opening of the housing.

14. The electronic device of claim 12, wherein the at least one magnetic body is arranged to face a partial area of the side surface of the housing, and
wherein the partial area of the side surface, is different from a partial area of the side surface of the housing where the connection terminal is formed.

15. The electronic device of claim 11, wherein the connection terminal protrudes from the side surface of the housing.
